# EUROPEAN PATENT SPECIFICATION

(11) **EP 1 266 623 B1**
(45) Date of publication and mention of the grant of the patent: **23.02.2005**
(21) Application number: 00911318.4
(22) Date of filing: 23.03.2000
(51) Int. Cl.: A61B 5/00

(54) **DIAGNOSIS DEVICE AND TREATMENT DEVICE**
DIAGNOSTISCHES GERÄT UND THERAPEUTISCHES GERÄT
DISPOSITIF DE DIAGNOSTIC ET DISPOSITIF DE TRAITEMENT

(43) Date of publication of application: 18.12.2002
(73) Proprietor: Sakamoto, Noriyasu, Tokyo 179-0076 (JP)
(72) Inventor: Sakamoto, Noriyasu, Tokyo 179-0076 (JP)
(74) Representative: von Hellfeld, Axel, Dr. Dipl.-Phys.,
(86) International application number: PCT/JP2000/001788
(87) International publication number: WO 2001/070113

(56) References cited:
- EP-A- 0 922 431
- JP-A- 4 180 730
- JP-A- 7 313 494
- JP-A- 9 505 760
- JP-A- 59 183 728
- US-A- 4 320 767
- US-A- 4 425 920
- US-A- 4 576 181
- US-A- 5 520 190
- 'Houshasen chiryou to CT' SHUUJUNSHA 20 December 1982, pages 22 - 35, XP002946970

## Description

### TECHNICAL FIELD

The present invention relates to devices for diagnosing and treating internal diseases, allergies, nervous disorders, etc.

### BACKGROUND ART

Up until now, the following results of researches have been published on the relationship between stimuli given to the five senses of a human being and responses of the human body to the stimuli.

In 1921, S.V. Kulakov, a Russian scientist, discovered that red stimulates the sympathetic nerve of the autonomous nervous system whilst blue stimulates the parasympathetic nerve (See Table 1).

In 1966, Paul Nogier, a French M.D., found that the pulse rate fluctuates due to stimuli such as electromagnetic field, sound, electricity and light, and named his finding "Blood Vessel-Autonomous Nerve Reflex" to report it at a medical conference.

In 1974, Dr. J.J. Platt and Dr. J. Sique discovered that specific colors affect the state of mind, breathing speed, pulse rate and blood pressure.

In 1975, M.D. Jacob Liberman reported at an eyesight measurement meeting held in Miami, U.S.A. that there is a correlation between light, color, eyesight and effects of treatment.

In 1979, American Drs. Martinek and Beredine reached, from their individual researches, the same conclusion below:
Some colors of light can improve the effect of internal enzymes as much as 500%; and
Some colors of light can increase the reaction rate of the internal enzymes, activate or deactivate them and affect the movement of substance passing through the cell membrane.

The above results of research are supported by Table 1.

Table 1 shows changes in the body temperature made after red and light blue images are each shown to a subject for 5 minutes.

The test results clearly indicate that the body temperature rises by showing the red image, whilst it falls by showing the light blue one.

It is thus proven that there is a strong relationship between stimuli such as light given to a human body and responses of the human body thereto. However, no such a technique have yet been developed that utilizes the above relationship for checkup and treatment of the human diseases.

Proposed in Japan Patent Laid-open Pub. No. Hei10-179581 for example is a remote diagnosis device using a communication line, etc.

In the conventional remote checkup devices, however, ultrasonic units and other units are placed on the user side to transfer data to the checker. Therefore, a subject must hold the ultrasonic unit on his/her own for the checkup with the device.

Under such a condition, remote treatment cannot be made even if remote checkup is possible.

It is therefore the object of the present invention firstly to provide a diagnosis device that utilizes the relationship between stimuli such as light given to a human body and the response of the human body thereto (blood vessel-autonomous nerve reflex), secondly to enable remote diagnosis requiring no special units other than a terminal and a measuring unit on the user (subject or patient) side, and thirdly to enable remote treatment.

### DISCLOSURE OF THE INVENTION

The invention defined in claim 1 provides a diagnosis device that comprises a checkup signal output means to output a checkup signal to a user terminal; a user terminal to input the checkup signal; a sample output means that loads the user (subject) with a checkup sample corresponding to the checkup signal; a measuring means to measure the vascular reflex of the user by sensing response to the loaded checkup sample; and a user-side recording means to record measurement values obtained by the measuring means. The diagnosis device is designed to diagnose the symptoms of the user on the basis of changes in the measurement values.

The checkup signal output from the host computer to the user terminal is a signal that, by itself, stimulates the five senses of the user to induce the vascular reflex of the user, or a signal that triggers loading the user with a checkup sample that stimulates the five senses of the user through the user-side equipment.

Such typical signals that stimulate the five senses of the user by themselves are music and other sound (including low-frequency waves and ultrasonic sound) , or images and other light (including laser beams and invisible rays) and electromagnetic waves. Sound and light signals are converted into checkup samples in an analog form on the user terminal, and those samples can be directly loaded into the user through the output equipment such as the speaker and monitor on the user side.

Such a signal that stimulates the five senses of the user through the equipment on the user side is, for example, an aroma indication signal. In this case, by putting various aromas in containers with a lid and setting them so that the container with the lid corresponding to the signal opens when a signal is received, the user can sense the aroma corresponding to the signal.

The checkup samples refer to information that stimulates the five senses of the user, and more specifically, they are the above sound, light, electromagnetic field and aroma.

The sample output means is a devise that transmits the checkup sample to the user in such a form that the five senses of the user can feel it. More specifically, if the sample is sound, color or aroma, the output means is a speaker (including earphones), monitor or aroma in a container, respectively.

The vascular reflex refers to chemical change in the blood generated when light, images, sound, electromagnetic field or aroma stimulates the five senses of a human body.

When the five senses are stimulated as above, catecholamine, dopamine, adrenaline, noradrenaline, acetylcholine esterase, etc. in the blood change to cause chemical change in the blood. Information on this chemical change is transmitted to the brain and all the arteries, resulting in blood vessel contraction and other phenomena. Therefore, the above vascular reflex can be detected and measured by measuring the brain waves and pulse (pulse rate, pulse waves, etc.).

The measuring means is a tool to measure the response (vascular reflex) of the user to the checkup sample. The vascular reflex appears in the brain waves, pulse rate, pulse waves, pulse, blood pressure, heart sound, the moisture of the skin (especially palm) , the pupils of the eyes, breathing, muscles, body temperature or blood circulation. Therefore, any instrument that can measure those will do.

The recording means on the checker side is a tool to record data on the vascular reflex sent from the user terminal. It records them into the host computer, records them as analog data on a recording paper and displays them temporarily on the monitor.

. The diagnosis based on vascular reflex data is made by a doctor, and it can also be made automatically by a computer equipped with a program to compare normal condition data and vascular reflex data.

An embodiment of the invention provides a diagnosis device that comprises a checker-side host computer to record checkup signal data for use in checkup and to output a checkup signal to a user terminal; a user terminal connected to the host computer, a signal output means to load the user with a checkup signal input to the user terminal as a checkup sample; a measuring means to measure the vascular reflex of the user by sensing the loaded signal; and a checker-side recording means to record measurement values obtained by the measuring means. The diagnosis device is designed to diagnose the symptoms of the user on the basis of changes in the measurement values.

As for the method of connecting the host computer on the checker side and the user terminal, any of cable connection, connection via host computer communication and connection via the Internet will do.

This means that in this invention it is not necessary for the checker and the user to be remote from each other and that they may be in the same hospital.

An embodiment of the invention is a device made by further providing the checker side with a comparing means to compare measurement values of a loaded user sent from the user terminal with the normal values; and with a diagnosis output means to output the result of symptom diagnosis by checking the result of the comparison against the symptom data pre-recorded by the checker-side host computer.

The normal values are input to the host computer on the checker side from the user terminal, together with data on a loaded user, as brain wave and pulse wave data obtained before and after the user is loaded with a checkup sample.

The checker-side host computer is equipped with a function to compare and calculate normal values and measurement values of a loaded user. It also records the symptom data against which the results of comparison are checked.

The symptom data gives the symptoms corresponding to the differences in each checkup sample between the normal values and the measurement values of a loaded user.

The invention defined in claim 6 uses, as a checkup signal, light that has penetrated through substance considered as a potential allergen.

According to this invention, the user develops the vascular reflex to the allergen, and therefore, the allergen can be identified without conducting the so-called badge test.

An embodiment of the invention is a treatment device further comprising a treatment signal transmitting means to retrieve treatment signal data pre-recorded in the checker-side host computer and to transmit it to the user terminal, based on output signals of the results of symptom diagnosis from the diagnosis output means.

The treatment signal data recorded by the user-side host computer is recorded together with their corresponding symptom diagnosis results. When a symptom diagnosis result is obtained, the treatment signal data corresponding to the result is identified and transmitted to the user terminal.

It is desirable that the treatment signal should be the same kind of signal as the checkup signal. More specifically, if the checkup signal is sound or color, the treatment signal should also be sound or color, respectively. By this method, the user can obtain the treatment signal using the same kind of equipment as that on the checker side.

According to this invention, the user's condition can be diagnosed on the basis of the vascular reflex data obtained from the checkup signal output from the checker side.

If sound or light is used as a checkup signal, the diagnosis can be made with only conventional equipment such as a speaker or a monitor. And if the host computer on the checker side is loaded with treatment data, the treatment can also be made automatically by inputting data from the user.

### BRIEF DESCRIPTION OF DRAWINGS

Fig. 1 is a block diagram that shows the configuration of the diagnosis device in this invention;
Fig. 2 is a block diagram that shows the configuration of the treatment device in this invention;
Fig. 3 is a drawing that shows an example of the vascular reflex (pulse wave) caused by white light; and
Fig. 4 is a drawing that shows the pulse wave responding to an allergen.

### BEST MODE FOR CARRYING OUT THE INVENTION

An embodiment of the present invention will now be described by way of an example in which the allergen is identified by measuring the pulse wave for vascular reflex, using, as a checkup signal, white light that has penetrated through substance considered as a potential allergen.

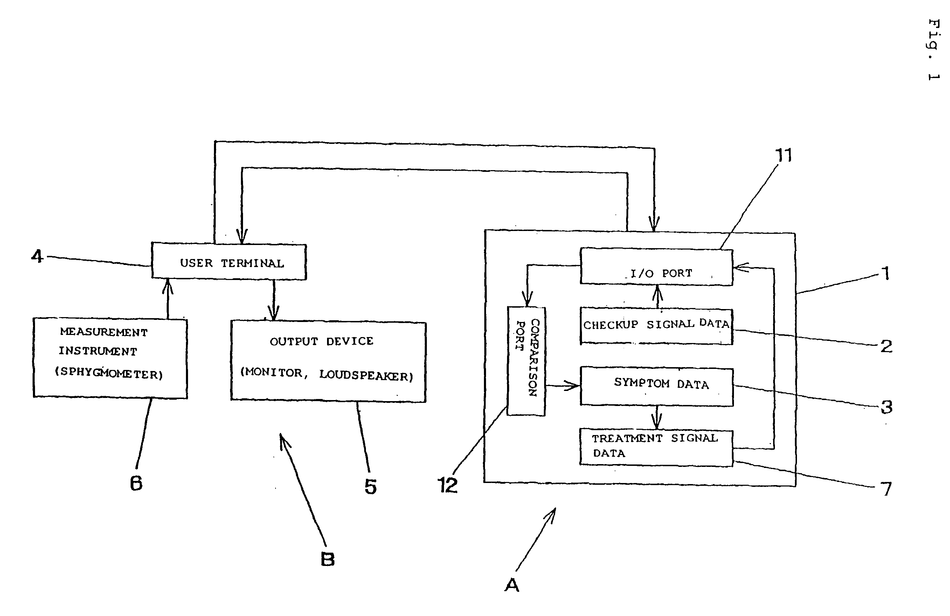

Fig. 1 is a drawing that shows the entire configuration, and "A" refers to the checker side while "B" refers to the user side.

A host computer 1 connected with the Internet or other communication line is installed on the checker side A.

The host computer 1 stores the following data:

Checkup signal data 2 in the form of data on white light that has penetrated through substance considered as a potential allergen (hereinafter referred to as "allergen data").

This allergen data is digitally recorded data on white light that has penetrated through substance considered a potential allergen which is placed between two transparent plates (transparent rubber filters are used). For example, a CCD camera can be used for digital recording.

Symptom data 3 in the form of data giving the results of comparing the normal pulse wave of the user and pulse wave upon vascular reflex together with their corresponding allergens.

The user terminal 4 connected to the host computer 1 via the Internet or other communication line and a monitor 5 connected to the user terminal 4 are installed on the user side B. Checkup signal data input into the user terminal 4 is displayed in an analog form as a checkup sample (light) on the monitor 5.

A sphygmometer 6 for measuring the pulse wave is connected to the user terminal 4 so as to output the pulse wave of the user to the host computer 1 via the user terminal 4.

When a plurality of allergen data is output to the user terminal 4 through an I/O port 11 from the checker-side host computer 1 by using the thus configured apparatus, light corresponding to each allergen data is displayed on the monitor 5.

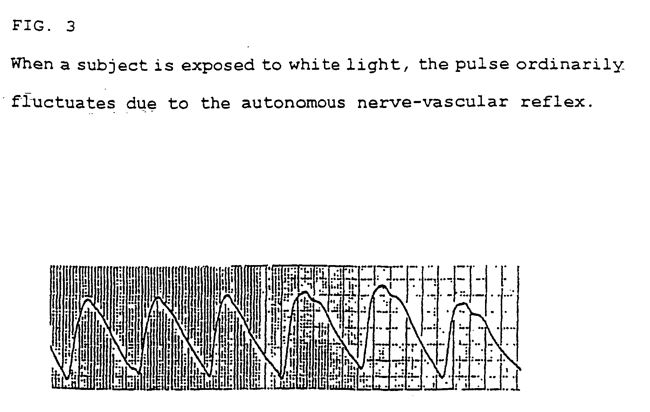

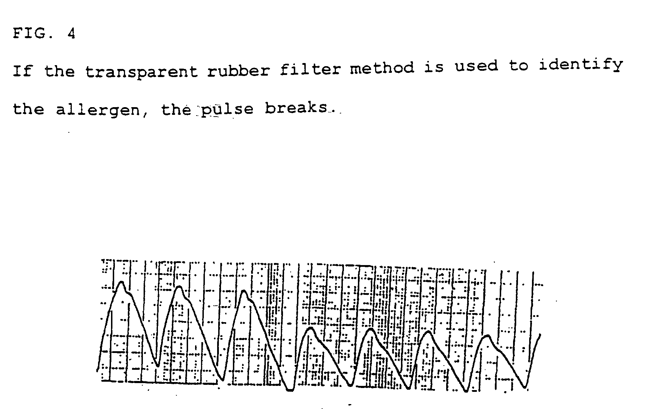

When the user puts on the sphygmometer 6 and views the monitor 5, the user develops vascular reflex and changes the pulse wave due to the stimulation of the light displayed on the monitor 5. (If there is no change, the user may possibly have a disease other than allergy. To be described later.) When the user receives the light that has penetrated though substance considered a potential allergen corresponding to the allergen peculiar to the user, blood vessel flex appears clearly and the pulse wave breaks. (See Figs. 3 and 4.)

The response of the user to each light is output from the user terminal 4 to the host computer 1 on the checker side in which the response is compared with the normal pulse wave. When the response corresponding to the allergen appears, the allergen is retrieved from symptom data 3 and identified on the basis of the response data.

Table 2 shows the diagnosis results of this invention and a badge test at a hospital, with the same sample used in both cases, which identify the allergen.

There is a slight difference between them, which falls within the allowable range of error. Therefore, the table proves the effectiveness of this invention.

An embodiment of a treatment device of this invention will then be described. The configuration of the equipment is the same as that in the above embodiment.

The host computer 1 stores the following data:

Checkup signal data 2 in the form of color data.

Symptom data 3 in the form of data giving the results of comparing the normal pulse wave of the user and that of the vascular reflex together with their corresponding colors.

Treatment signal data 7 in the form of data containing effective colors and sounds for treatment of symptoms recorded as the symptom data 3.

As set forth earlier, it is found that if the user has a disease, the vascular reflex does not respond to some particular colors (0 response) . For example, the 0 response to orange color, red color, blue color, bitter orange color, green color and purple color means neurosis, psychological trouble, eczema, insomnia and epilepsy, respectively.

It is also confirmed that particular colors contribute to the treatment depending on the disease. For example, if a patient suffering from amblyopia is loaded with clear white color, the eyesight is drastically improved, though exposing reddish purple or other colors barely improves the vision. (See Table 3.)

When a plurality of color data is output in sequence to the user terminal 4 from the I/O port 11 of the checker-side host computer 1 by using the thus configured apparatus, the light (color) corresponding to each color data is displayed on the monitor 5.

When the user puts on the sphygmometer 6 and views the monitor 5, the user develops vascular reflex and changes the pulse wave due to the stimulation of the light displayed on the monitor. If the user has a disease, vascular reflex does not occur when exposed to light of particular colors.

The response (pulse wave data) of the user to each light is output to the checker-side host computer 1 from the user terminal 4 and is compared with the normal pulse wave at a comparison port 12. If a color that does not cause the vascular reflex is detected, the disease corresponding to the color can be identified.

When the disease is identified as explained above, the treatment signal data is retrieved from the name of the identified disease as a key word. Only color (including images) or sound (including music) or combination of both should be used as a treatment signal data.

When the treatment signal data corresponding to the disease is retrieved, it is output to the user terminal 4 from the I/O port 11 of the host computer 1. Then, it is output from the output device as light or sound on the monitor or speaker, respectively, stimulating the user.

With this stimulation, the treatment is carried out.

Table 4 shows the effect on a user suffering from insomnia by transmitting the treatment signal data to the user, using the device in this invention. It indicates that the treatment is very effective.

Although the above embodiment includes the device for measuring vascular reflex to perform the checkup, the following embodiment is directed to only the treatment. It is therefore suitable for remote treatment of patients who have already known their diseases.

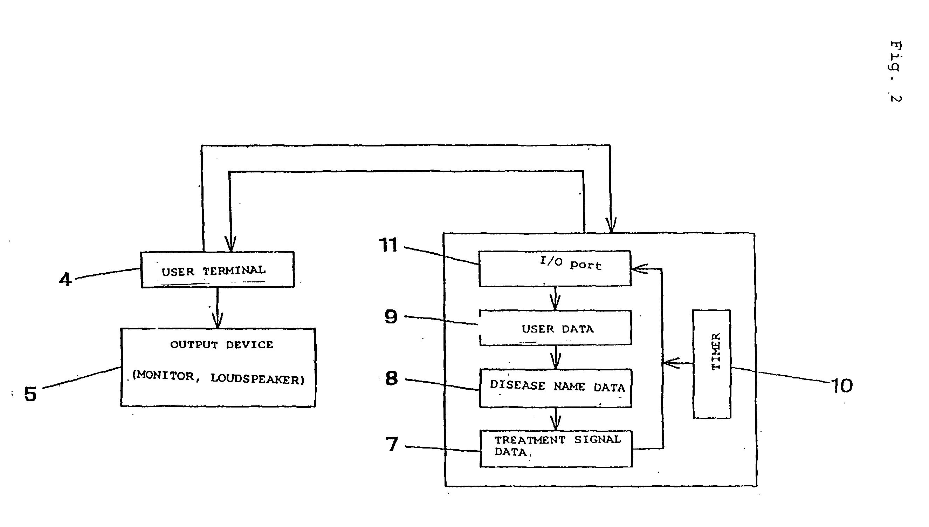

As shown in Fig. 2, the treatment device of this embodiment comprises a host computer 1 on the medical staff side, a user terminal 4 on the patient side connected with the host computer, and an output unit 5 such as a monitor and a loudspeaker.

The host computer 1 stores treatment signal data 7, disease name data 8, user data 9 and a timer 10.

The treatment signal data is stored in the form of only color (including images) or sound (including music) or combination of both and can be retrieved with the disease name as a key word.

The user data 9 consists of the names of patients registered for treatment with this device, the names of the diseases of those patients and the history of treatment with this device.

The timer 10 is used to set proper treatment duration, and when the designated period has elapsed, it stops the output of the treatment signal data.

To receive treatment with this device, the patient is required to make access to the host computer 1 from his/her own user terminal 5. When the patient is connected, start screen data is transmitted from the host computer 1 to the user terminal 5, and is displayed on the monitor.

The start screen asks for the input of the user' s name (patient's name) and the password. When the user gives them, the host computer 1 retrieves the user data 9 from the user's name as a key word, and identifies the name of the disease of the user. Then, the host computer retrieves treatment signal data 7 from the identified disease name as a key word, and outputs the treatment signal data suitable to treat the disease of the user.

The treatment signal data output from the host computer 1 is input into the user terminal 4 and is output from the monitor or loudspeaker to stimulate the user. When a predetermined duration has elapsed, one session of the treatment is completed as the output of the treatment signal data is stopped by the timer 10.

Table 5 shows the effect on users suffering from amblyopia or other diseases by showing them colors corresponding to their diseases, using this device.

In all the cases, the number of results that the conditions of those users have not been improved is very few. Therefore, the table proves the effectiveness of treatment by this invention.

In this invention, an ordinary personal computer and its accessory monitor or speaker are used as the user terminal and output unit such as a monitor and a loudspeaker. Therefore, no special device is required for the treatment.

Furthermore, the treatment signal data can be displayed as a background color on the monitor, enabling the user to receive treatment while operating the personal computer.

A cellular phone and interactive TV are also expected to serve as the user terminal and output equipment.

### INDUSTRIAL APPLICABILITY

According to the present invention, a disease can be identified on the basis of the principle of vascular reflex when the user is loaded with a checkup sample such as color from output equipment such as monitor. Therefore, the user (user) can check for and identify his/her disease by preparing only a personal computer and measuring instruments such as sphygmometer without going to the hospital.

The user (patient) can also receive treatment by preparing only a personal computer without going to hospital.

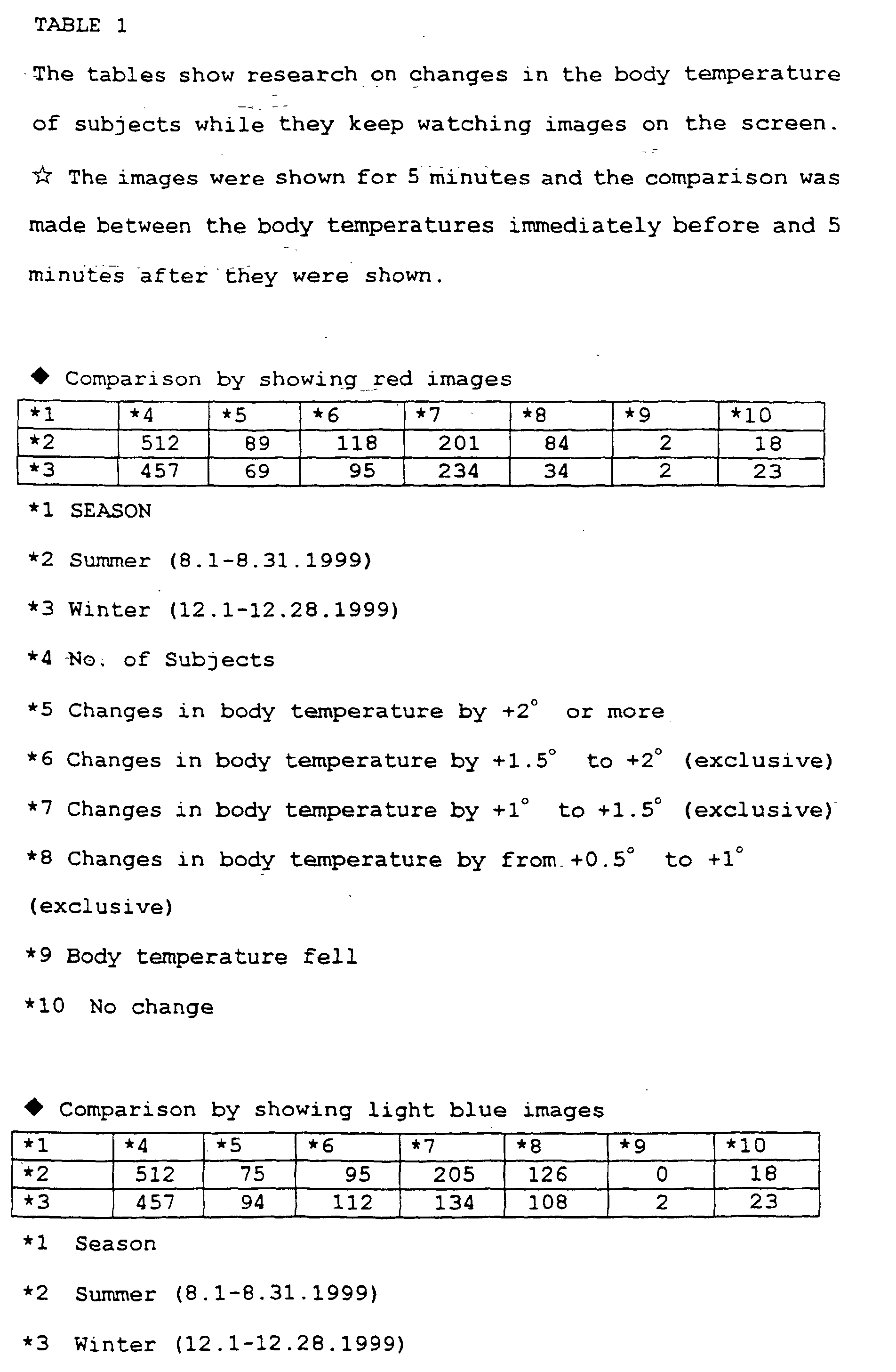

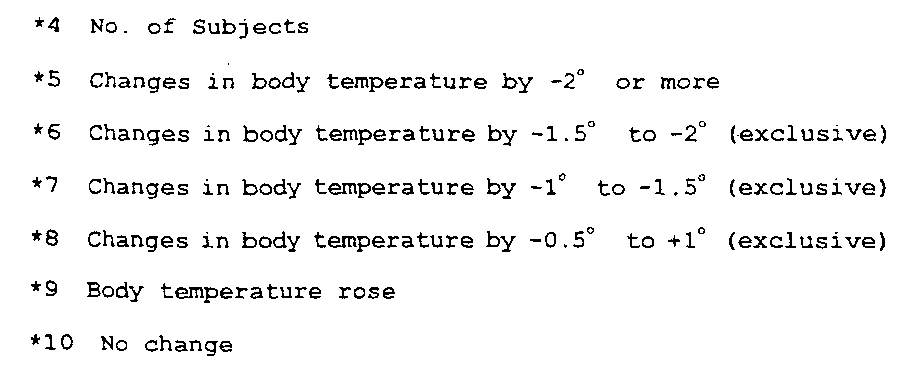

**TABLE 2**

| | | |
|---|---|---|
| Clinical example where the allergen was identified by showing subjects the light (exposing subjects to the image light) that penetrated through images placed between transparent rubber filters to find nonresistant substance | | |

| *1 | *2 | *3 |
|---|---|---|
| Tea | 71 | 70 |
| Coffee | 43 | 43 |
| Orange | 41 | 40 |
| Egg | 103 | 100 |
| Legumes | 95 | 89 |
| Pork | 76 | 72 |
| Beef | 63 | 61 |
| Chicken | 55 | 55 |
| Wheat, Bread, Pasta | 88 | 88 |
| Corn | 95 | 95 |
| Oil | 28 | 27 |
| Butter | 65 | 65 |
| Yogurt | 48 | 47 |
| Cream | 55 | 55 |
| Cheese | 52 | 52 |
| Wine | 21 | 20 |
| Sugar | 57 | 55 |
| Mushroom | 20 | 20 |
| Chocolate | 49 | 44 |
| Rice | 21 | 21 |
| Milk | 52 | 52 |
| Tofu(Bean Curd) | 73 | 70 |
| Fermented Soybeans | 69 | 69 |
| Mayonnaise | 55 | 54 |
| Confectionery | 184 | 169 |

| | | |
|---|---|---|
| *1 Nonresistant food shown as images | | |
| *2 Number of patients whose allergens were identified by the rubber filter method | | |
| *3 Number of cases where the rubber filter method was reconfirmed by identifying the allergens of patients at hospitals in Tokyo | | |

**TABLE 3**

| | | | |
|---|---|---|---|
| Table for comparing vision improvement by showing white color and other colors to patients suffering from amblyopia | | | |

| *1 | *2 | *3 | *4 |
|---|---|---|---|
| Clear white | 200 | 196 | 4 |
| Reddish purple | 200 | 11 | 189 |
| Navy blue | 200 | 0 | 200 |
| Indigo blue | 200 | 19 | 181 |
| Gray | 200 | 0 | 200 |

| | | | |
|---|---|---|---|
| *1 Kinds of colors shown as images | | | |
| *2 Number of Subjects | | | |
| *3 Number of patients whose eyesight was improved to 1.0 (20/20 vision) or better | | | |
| *4 Number of patients whose eyesight was not improved | | | |

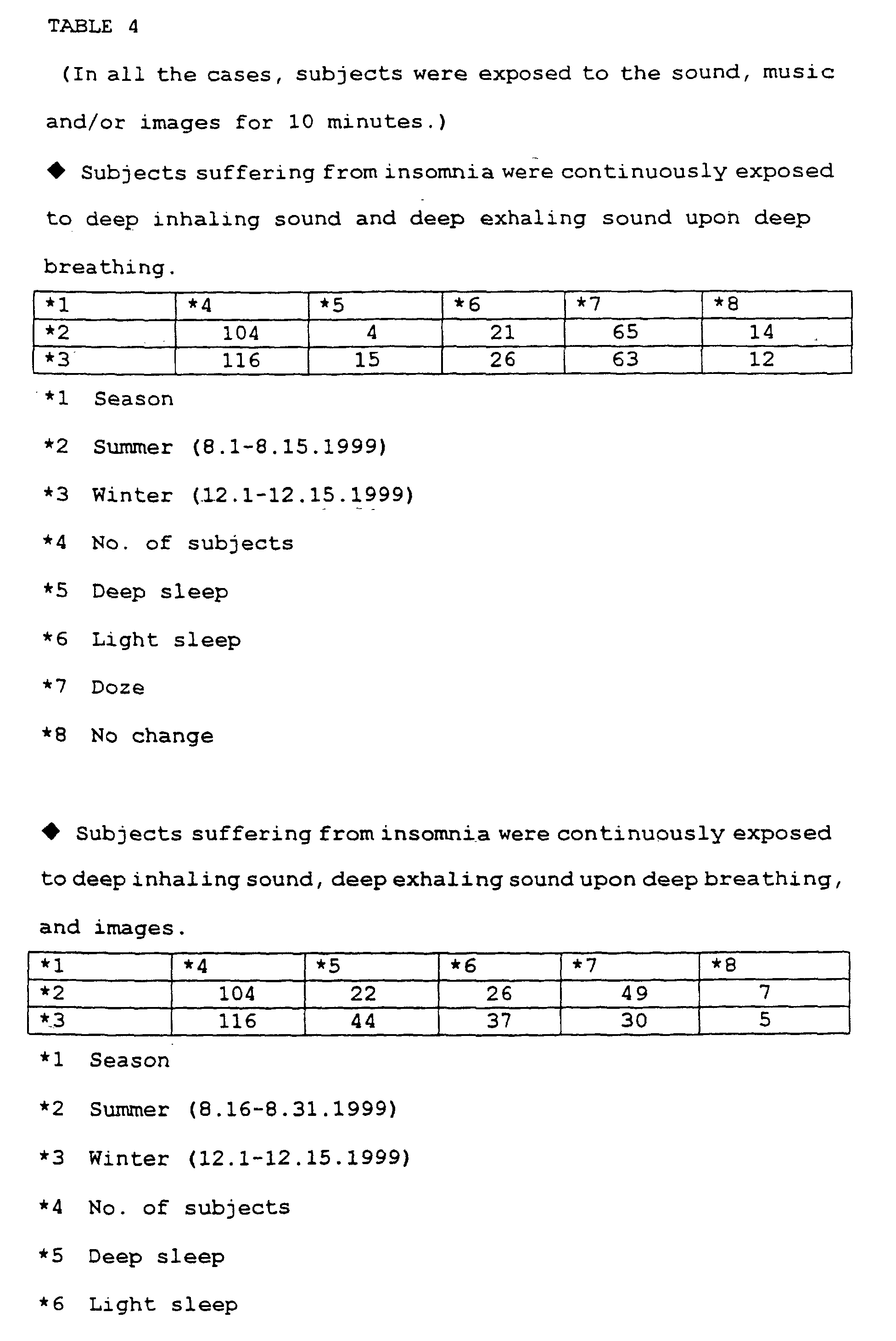

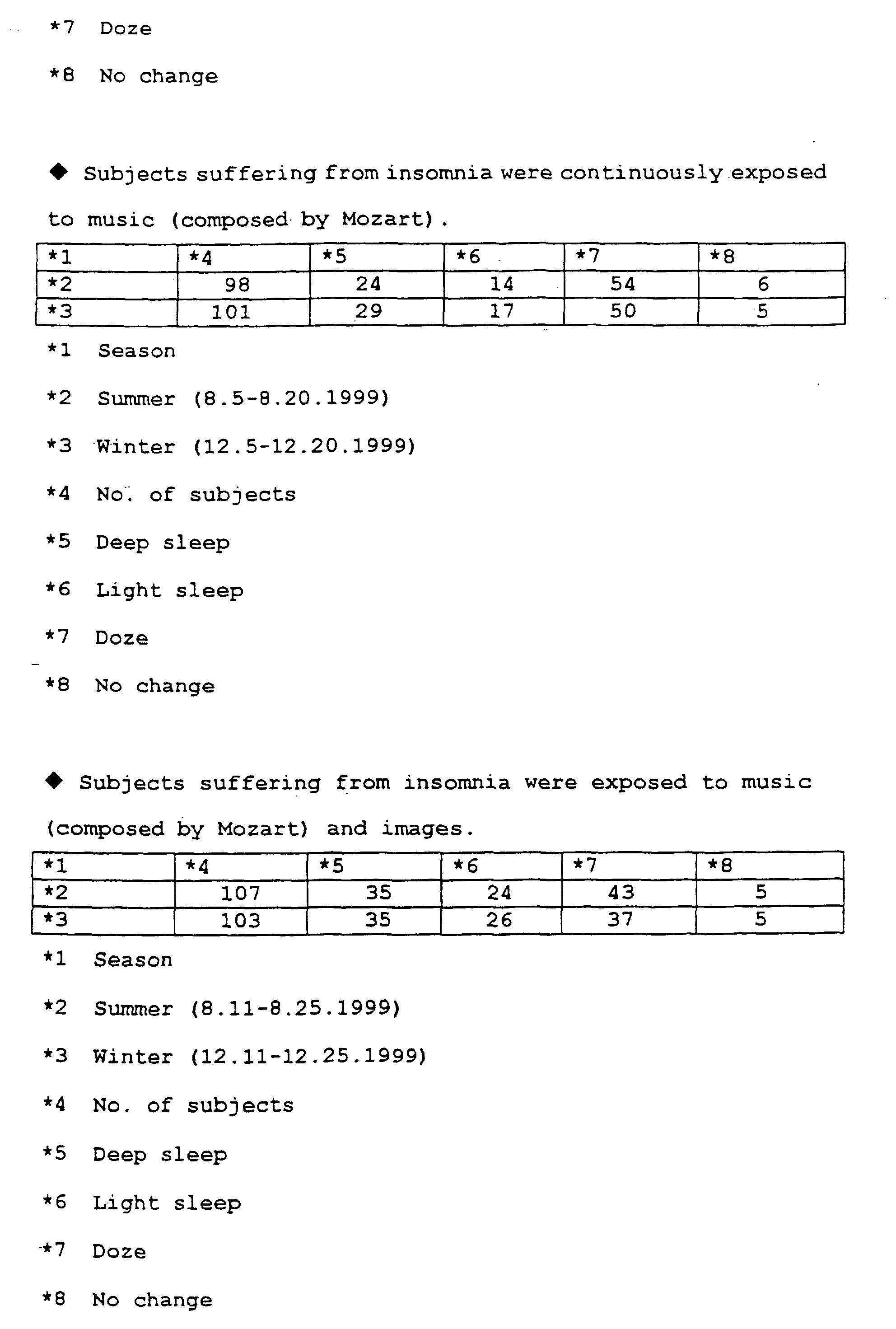

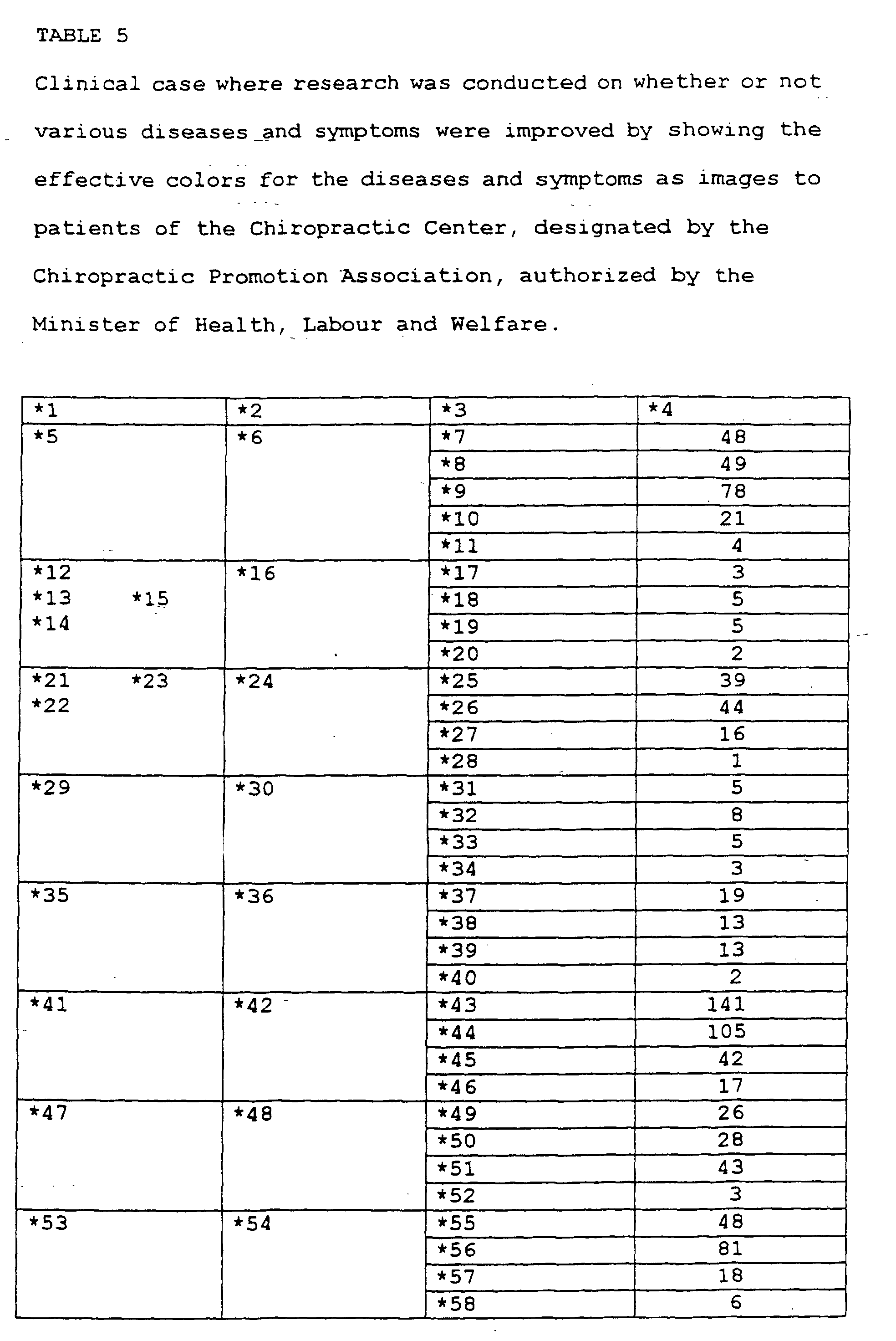

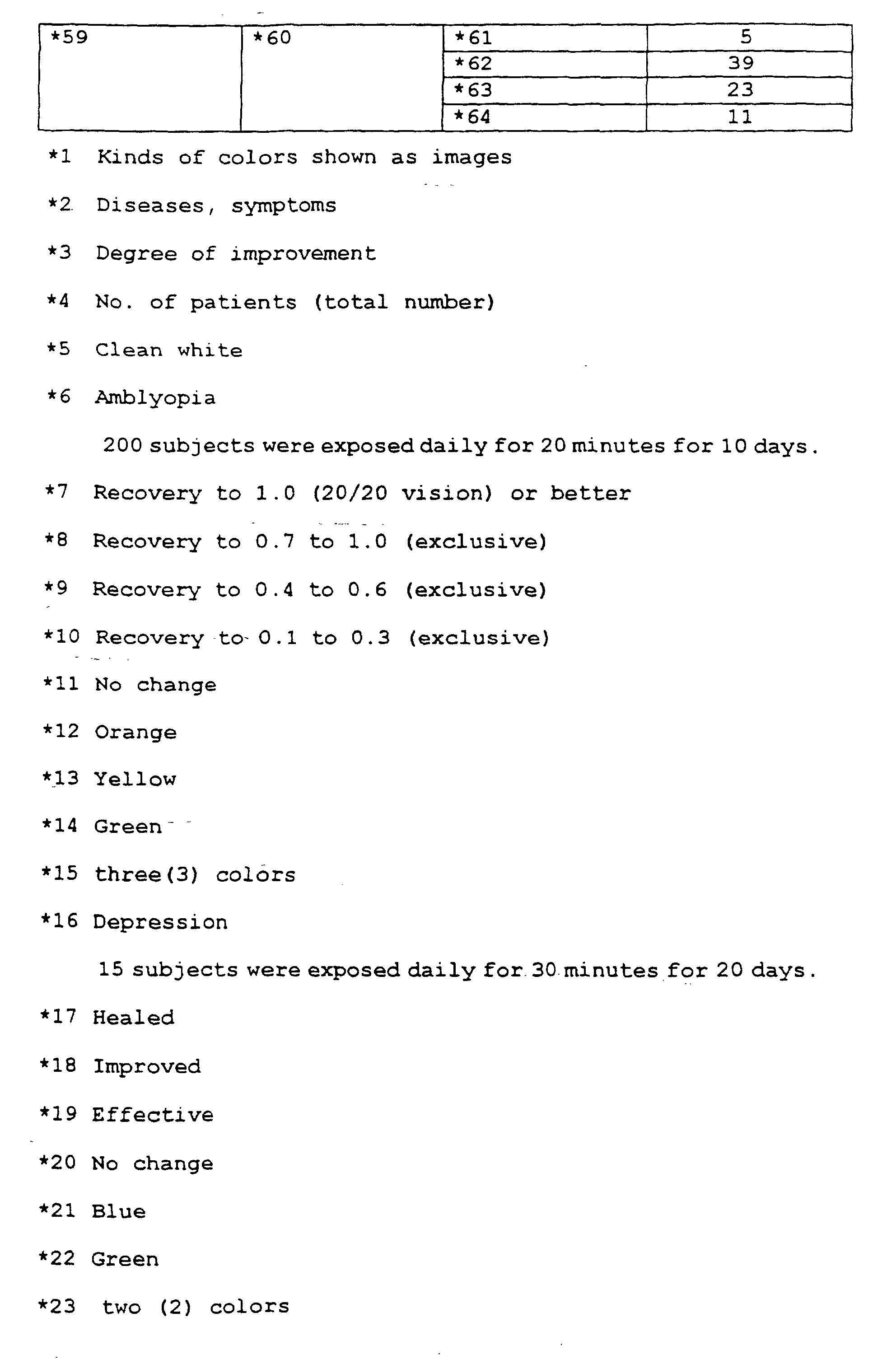

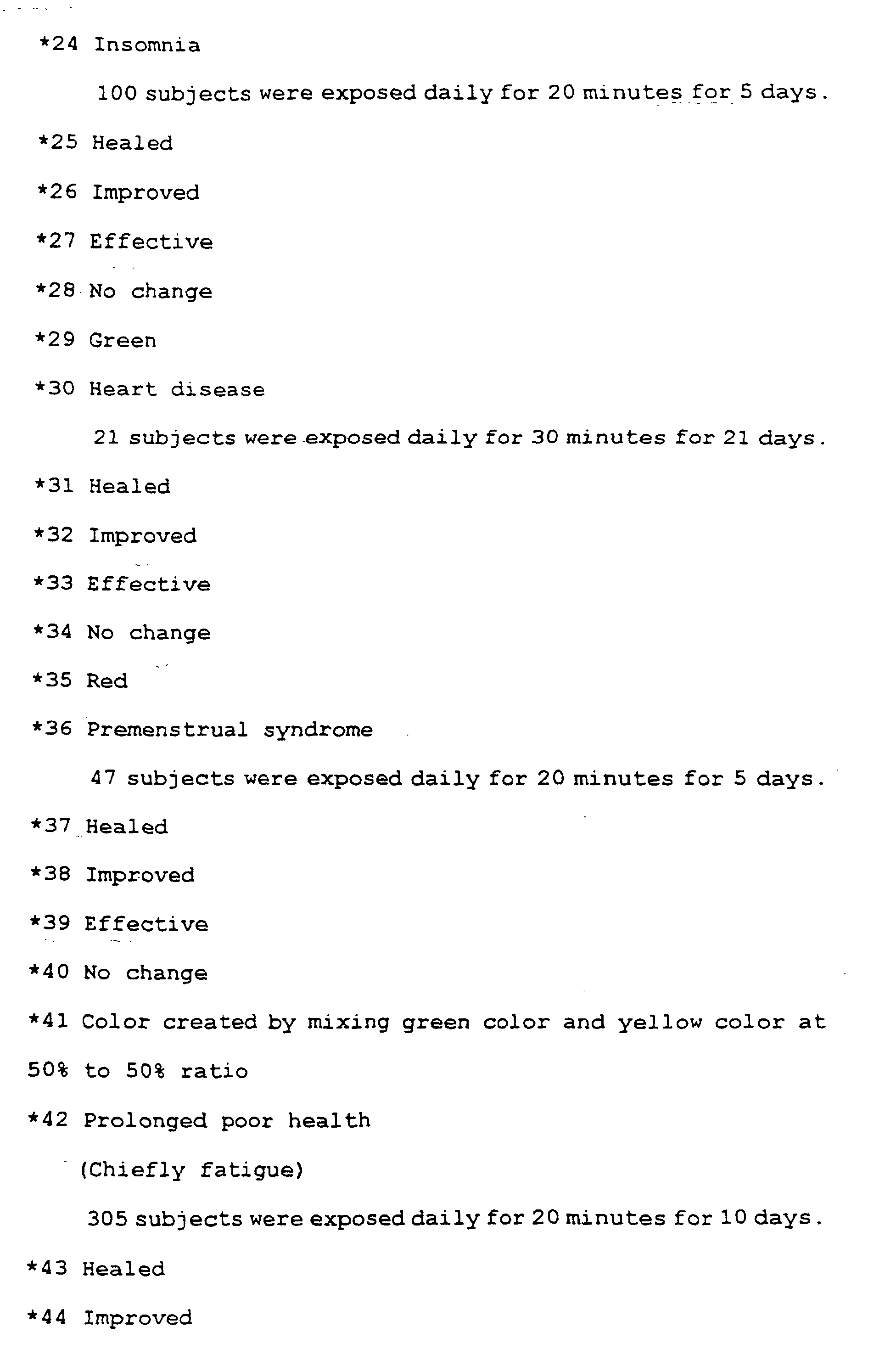

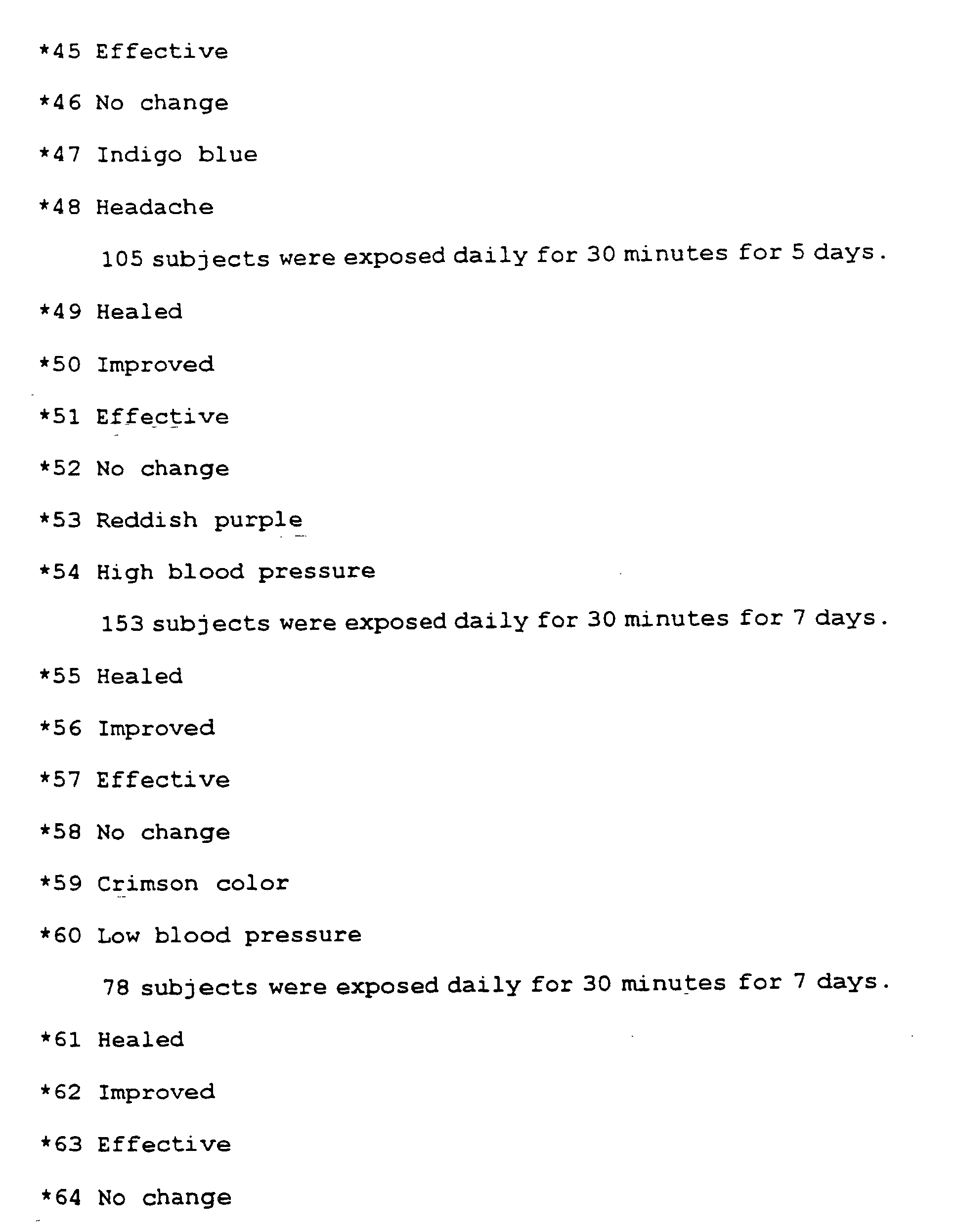

## Claims

1. A diagnosis device **characterised by**:
a checkup signal output means which outputs a checkup signal to a user, i.e. subject or patient, terminal;
a user terminal which inputs the checkup signal;
a signal output means which loads a user with a checkup sample corresponding to the checkup signal, or with the checkup signal input to the user terminal as a checkup sample;
a measuring means which measures the vascular reflex of the user referring to chemical change in the blood generated when light, images, sound, electromagnetic field or around stimulates the five senses of the human body, i.e. the blood vessel-autonomous nerve reflex, by sensing response to the loaded checkup sample or checkup signal; and
a checker-side recording means which records measurement values obtained by the measuring means;
wherein the condition of the user is diagnosed on the basis of the changes in the measurement values.

2. A diagnosis device according to claim 1, wherein
the checkup signal output means is a checker-side host computer which records a checkup signal for use in checkup and outputs the checkup signal to a user terminal connected to the checker-side host computer.

3. A diagnosis device according to claim 2, further comprising:
a comparison means which compares the measurement values with normal values; and
a diagnosis output means which outputs symptom diagnosis results by checking the comparison results against symptom data pre-recorded in the checker-side host computer.

4. A diagnosis device according to any one of claims 1 to 3, wherein
the checkup signal is color and the signal output means is a monitor.

5. A diagnosis device according to any one of claims 1 to 3, wherein
the checkup signal is sound and the signal output means is a loudspeaker.

6. A diagnosis device according to any one of claims 1 to 3, wherein
the checkup signal is light that has penetrated through substance considered as a potential allergen, and the signal loading means is a monitor.

7. A diagnosis device according to any one of claims 1 to 3, wherein
the measuring means is a sphygmometer.

8. A diagnosis device according to claim 3, further comprising:
a treatment signal transmitting means which retrieves treatment signal data pre-recorded in the checker-side host computer, on the basis of an output signal of the diagnosis output means, and transmits the retrieved data to the user terminal.

9. A treatment device **characterised by**:
a diagnosis device according to claim 3; and
a treatment signal transmitting means to retrieve treatment signal data pre-recorded in the checker-side host computer and to transmit said treatment signal data to the user terminal, based on output signals of the results of symptom diagnosis from the diagnosis output means.

10. A treatment device according to claim 9, wherein
the checker-side host computer is a medical-staff-side host computer which records treatment signal data for use in treatment, that corresponds to the symptom of a patient.

11. A treatment device according to claim 10, comprising:
a signal output means which loads a user with the treatment signal transmitted to the user terminal, wherein the symptoms of the user are treated by transmitting the treatment signal to the user terminal.

## Patentansprüche

1. Ein diagnostisches Gerät, **gekennzeichnet durch**:
eine Prüfsignalausgabevorrichtung, welche ein Prüfsignal an ein Terminal eines Benutzers, d.h. eine Person oder einen Patienten, ausgibt;
ein Benutzerterminal, welches das Prüfsignal eingibt;
eine Signalausgabevorrichtung, welche einen Benutzer mit einer Prüfprobe entsprechend dem Prüfsignal oder mit dem Prüfsignal beaufschlagt, welches dem Benutzerterminal als Prüfsignal eingegeben wird;
eine Messvorrichtung, welche den Vaskulärreflex des Benutzers, zurückzuführen auf eine chemische Änderung in dem Blut, die erzeugt wird, wenn Licht, Bilder, Geräusch, ein elektromagnetisches Feld oder Aroma die fünf Sinne des menschlichen Körpers stimuliert, d.h. den blutgefäßautonomen Nervenreflex misst, indem ein Ansprechen auf die aufgebrachte Prüfprobe oder das Prüfsignal sensiert wird; und
eine überprüferseitige Aufzeichnungsvorrichtung, welche Messwerte aufzeichnet, die von der Messvorrichtung erhalten wurden; wobei
der Zustand des Benutzers auf der Grundlage der Änderungen in den gemessenen Werten diagnostiziert wird.

2. Ein diagnostisches Gerät nach Anspruch 1, wobei die Prüfsignalausgabevorrichtung ein prüferseitiger Host-Computer ist, der ein Prüfsignal zur Verwendung bei der Prüfung aufzeichnet und das Prüfsignal an ein Benutzerterminal ausgibt, welches mit dem prüferseitigen Hostcomputer verbunden ist.

3. Ein diagnostisches Gerät nach Anspruch 2, weiterhin mit:
einer Vergleichsvorrichtung, welche die gemessenen Werte mit normalen Werten vergleicht; und einer Diagnoseausgabevorrichtung, welche Symptomdiagnoseergebnisse durch Prüfen der Vergleichsergebnisse mit Symptomdaten ausgibt, welche vorab in dem prüferseitigen Host-Computer aufgezeichnet werden.

4. Ein diagnostisches Gerät nach einem der Ansprüche 1 bis 3, wobei das Prüfsignal Farbe ist und die Signalausgabevorrichtung ein Monitor ist.

5. Ein diagnostisches Gerät nach einem der Ansprüche 1 bis 3, wobei das Prüfsignal ein Geräusch ist und die Signalausgabevorrichtung ein Lautsprecher ist.

6. Ein diagnostisches Gerät nach einem der Ansprüche 1 bis 3, wobei das Prüfsignal Licht ist, welches durch eine Substanz hindurch getreten ist, welches als mögliches Allergen betrachtet wird und die Signalaufbringvorrichtung ein Monitor ist.

7. Ein diagnostisches Gerät nach einem der Ansprüche 1 bis 3, wobei die Messvorrichtung ein Pulsmesser ist.

8. Ein diagnostisches Gerät nach Anspruch 3, weiterhin mit:
einer Therapiesignal-Übertragungsvorrichtung, welche Therapiesignaldaten, die in dem prüferseitigen Host-Computer vorab aufgezeichnet werden, auf der Grundlage eines Ausgangssignals der Diagnose-Ausgabevorrichtung erstellt und die erstellten Daten an das Benutzerterminal überträgt.

9. Ein therapeutisches Gerät, **gekennzeichnet durch**:
ein diagnostisches Gerät nach Anspruch 3; und
eine Therapiesignalübertragungsvorrichtung zur Erstellung von Therapiesignaldaten, die in dem prüferseitigen Host-Computer vorab aufgezeichnet werden und zur Übertragung der Therapiesignaldaten an das Benutzerterminal auf der Grundlage von Ausgangssignalen der Ergebnisse der Symptomdiagnose von der Diagnoseausgangsvorrichtung.

10. Ein therapeutisches Gerät nach Anspruch 9, wobei der prüferseitige Host-Computer ein klinikpersonalseitiger Host-Computer ist, der Therapiesignaldaten zur Anwendung bei der Therapie aufzeichnet, welche dem Symptom eines Patienten entspricht.

11. Ein therapeutisches Gerät nach Anspruch 10, mit:
einer Signalausgabevorrichtung, welche einen Benutzer mit einem Therapiesignal versorgt, welches dem Benutzerterminal übertragen wird, wobei die Symptome des Benutzers durch Übertragung des Therapiesignals an das Benutzerterminal behandelt werden.

## Revendications

1. Dispositif de diagnostic **caractérisé par** :
un moyen de sortie de signal de contrôle qui délivre en sortie un signal de contrôle à un terminal utilisateur, c'est-à-dire un sujet ou un patient ;
un terminal utilisateur qui reçoit le signal de contrôle ;
un moyen de sortie de signal qui charge un utilisateur avec un échantillon de contrôle correspondant au signal de contrôle ou avec le signal de contrôle délivré au terminal utilisateur en tant qu'échantillon de contrôle ;
un moyen de mesure qui mesure le réflexe vasculaire de l'utilisateur en se référant à un changement chimique dans le sang généré lorsque de la lumière, des images, du son, un champ électromagnétique ou un arôme stimule les cinq sens du corps humain, c'est-à-dire les réflexes du système nerveux autonome pour les vaisseaux sanguins, en détectant la réponse au signal de contrôle ou à l'échantillon de contrôle chargé ; et
un moyen d'enregistrement côté contrôleur qui enregistre les valeurs de mesure obtenues par le moyen de mesure ;
dans lequel l'état de l'utilisateur est diagnostiqué en se basant sur les changements des valeurs de mesure.

2. Dispositif de diagnostic selon la revendication 1, dans lequel le moyen de sortie de signal de contrôle est un ordinateur hôte côté contrôleur qui enregistre un signal de contrôle destiné à être utilisé pour le contrôle et délivre en sortie le signal de contrôle à un terminal utilisateur connecté à l'ordinateur hôte côté contrôleur.

3. Dispositif de diagnostic selon la revendication 2, comprenant en outre :
un moyen de comparaison qui compare les valeurs de mesure à des valeurs normales ; et
un moyen de sortie de diagnostic qui délivre en sortie des résultats de diagnostic de symptôme en contrôlant les résultats de la comparaison par rapport à des données pré-enregistrées dans l'ordinateur hôte côté contrôleur.

4. Dispositif de diagnostic selon l'une quelconque des revendications 1 à 3, dans lequel le signal de contrôle est une couleur et le moyen de sortie de signal est un écran.

5. Dispositif de diagnostic selon l'une quelconque des revendications 1 à 3, dans lequel le signal de contrôle est un son et le moyen de sortie de signal est un haut-parleur.

6. Dispositif de diagnostic selon l'une quelconque des revendications 1 à 3, dans lequel le signal de contrôle est une lumière qui pénètre dans une substance considérée comme un allergène potentiel et le moyen de chargement de signal est un écran.

7. Dispositif de diagnostic selon l'une quelconque des revendications 1 à 3, dans lequel le moyen de mesure est un sphygmomètre.

8. Dispositif de diagnostic selon la revendication 3, comprenant en outre :
un moyen de transmission de signal de traitement qui récupère des données de signal de traitement pré-enregistrées dans l'ordinateur hôte côté contrôleur, en se basant sur un
signal de sortie du moyen de sortie de diagnostic, et transmet les données récupérées au terminal utilisateur.

9. Dispositif de traitement **caractérisé par** :
un dispositif de diagnostic selon la revendication 3 ; et
un moyen de transmission de signal de traitement pour récupérer des données de signal de traitement pré-enregistrées dans l'ordinateur hôte côté contrôleur et transmettre lesdites données de signal de traitement au terminal utilisateur, en se basant sur les signaux de sortie des résultats du diagnostic de symptôme à partir du moyen de sortie de diagnostic.

10. Dispositif de traitement selon la revendication 9, dans lequel
l'ordinateur hôte côté contrôleur est un ordinateur hôte côté personnel médical qui enregistre des données de signal de traitement destinées à être utilisées dans un traitement qui correspond au symptôme d'un patient.

11. Dispositif de traitement selon la revendication 10, comprenant :
un moyen de sortie de signal qui charge un utilisateur avec le signal de traitement transmis au terminal utilisateur, dans lequel les symptômes de l'utilisateur sont traités en transmettant le signal de traitement au terminal utilisateur.
